# EUROPÄISCHE PATENTSCHRIFT

(11) **EP 0 934 921 B1**
(45) Veröffentlichungstag und Bekanntmachung des Hinweises auf die Patenterteilung: **09.01.2002**
(21) Anmeldenummer: 98102182.7
(22) Anmeldetag: 09.02.1998
(51) Int. Cl.: C07C 69/604, C07C 67/08, A61K 7/027, A61K 9/02

(54) **Wachse auf nativer Rohstoffbasis**
Waxes based on native raw material
Cires à base de matières premières natives

(43) Veröffentlichungstag der Anmeldung: 11.08.1999
(73) Patentinhaber: Chemtec Leuna Gesellschaft für Chemie und Technologie mbH, 06236 Leuna (DE)
(72) Erfinder: Klimek, Irene, Dipl.-Chem., 06122 Halle (DE); Becker, Karl, Dr., 06237 Leuna (DE); Brauer, Erhard, Dr., 06217 Knapendorf (DE)
(74) Vertreter: Schinke, Herbert, Dr. Dr., Patentanwaltskanzlei

(56) Entgegenhaltungen:
- DE-A- 2 551 173
- DATABASE WPI Week 7843 Derwent Publications Ltd., London, GB; AN 78-77552a XP002068711 & JP 53 108 917 A (NIPPON OILS AND FATS CO LTD) , 22.September 1978

## Beschreibung

Die Erfindung betrifft Wachse auf nativer Rohstoffbasis, wie sie insbesondere als Rohstoff für kosmetische und pharmazeutische Formulierungen und für verschiedene technische Anwendungen gebraucht werden, und ein Verfahren zu ihrer Herstellung.

Es ist bekannt, daß Erzeugnisse der dekorativen Kosmetik wie Augen- oder Lippenstifte oder Erzeugnisse der pharmazeutischen Industrie, z.B. Suppositorien, Wachsester unterschiedlicher Herkunft und Konsistenz enthalten.

Die Auswahl an geeigneten Wachsestern für kosmetische und pharmazeutische Formulierungen beschränkt sich im wesentlichen auf Naturwachse und deren Substitute wie Bienenwachs, Carnaubawachs, Candellilawachs oder chemisch hergestellte Wachsester, die durch Veresterung von in der Regel einwertigen Fettalkoholen mit Fettsäuren der Kettenlänge C12 bis C30 hergestellt werden.

Ein typischer Vertreter der chemisch hergestellten Wachsester ist das Cetylpalmitat.

Bienenwachs besitzt als klassischer kosmetischer und pharmazeutischer Rohstoff seit jeher ein starkes Interesse. Es wird zur Herstellung von Cremes, aber auch bei Formulierungen von Lippenstiften oder Suppositorien eingesetzt. Seine Klebrigkeit und Plastizität bewirken Flexibilität und Bruchfestigkeit der unter Verwendung von Bienenwachs formulierten Stifte. Für diese Anwendungen ist es den hergestellten Wachsestern weit überlegen. Chemisch gesehen ist Bienenwachs hauptsächlich ein Gemisch von Estern einer langkettigen aliphatischen Carbonsäure mit einem langkettigen einwertigen Alkohol, freien Wachssäuren und Kohlenwasserstoffen. Ein Gemisch von polaren Stoffen ist auch das pflanzliche Carnaubawachs.

Jedoch sind Naturwachse je nach Herkunft von schwankender Qualität, auch die nicht ausreichende Verfügbarkeit steht einem breiteren Einsatz entgegen. Es hat daher nicht an Bemühungen gemangelt, Wachse herzustellen, die die Konsistenz und die Eigenschaften wie Flexibilität, Klebrigkeit, Plastizität von Naturwachsen aufweisen.

In der DD 217 530 wird z. B. ein Kosmetikgrundwachs, bestehend aus einer Kombination aus 20 bis 50 % eines Vaselins, 30 bis 70 % eines Montanwachsraffinates und 5 bis 25 % eines Ethylenvinylester-Copolymeren, beschrieben.

Die DE-PS 28 56 277 schützt ein Gemisch aus Glyzerol Hydroxystearat, Cetylpalmitaten, mikrokristallinem Wachs und Trihydroxystearin. Dieses komponierte Bienenwachssubstitut wird hauptsächlich zur Konsistenzeinstellung von Stiften verwendet.

Trotz weitgehender anwendungstechnischer Ähnlichkeit sind bei einem Austausch solcher zubereiteten Wachse umfangreiche Labortests erforderlich. Oft ist es daher günstiger, von einem Rohstoff auszugehen, der ein geeignetes Kennwertprofil aufweist, um je nach gewünschter Konsistenz die Möglichkeit zu haben, Einzelkomponenten zur Feinabstimmung gezielt zuzusetzen.

Das in der DE-PS 28 56 277 mitverwendete mikrokristalline Wachs bzw. die Vaseline in der DD 217 530 sind zudem Produkte, die überwiegend aus dem Erdöl gewonnen werden und mit allen Nachteilen eines mineralölstämmigen Produktes behaftet sind (Hautreizungen und Unverträglichkeitserscheinungen).

Für Suppositorien ist ferner eine Grundmasse bekannt, die ein Gemisch aus Mono-, Di- und Triglyceriden gesättigter natürlicher Fettsäuren der Kettenlänge C12 bis C18 darstellt (Jahrbuch für den Praktiker 1988, Seite 66, Verlag für chemische Industrie, Ziolkowski KG, Augsburg).

Sie deckt im Eigenschaftsprofil jedoch nicht die weiteren Anwendungen, insbesondere dort, wo Flexibilität gewünscht ist.

Ziel ist es daher, eine Grundwachskomponente mit hoher Flexibilität und Plastizität zu entwickeln, die ausschließlich aus Rohstoffen auf Pflanzenölbasis hergestellt wird und als Basisprodukt für zubereitete Wachse für verschiedenste Anwendungen dienen kann. Das Wachs soll in ständig gleichbleibender Qualität und in ausreichender Menge zur Verfügung stehen.

Das Basiswachs soll als Konsistenzgeber für kosmetische und pharmazeutische Formulierungen, wie für Cremes, Sticks, Suppositorien, als Modellwachsbasis für zahnärztliche Labortechnik, aber auch für technische Anwendungen, z. B. als Wachskomponente für Schmelzkleber, Wachsmalstifte, Kitt- und Dichtungsmassen, und als Beschichtungskomponente für Nahrungsmittel(verpackungen) geeignet sein.

Diese Aufgabe wird erfindungsgemäß gelöst mit Wachsen auf nativer Rohstoffbasis, die zu mindestens 90 Gew.% aus dem Umsetzungsprodukt von
a) Glycerinmonoestern mit geradkettigen C14 bis C22-Fettsäuren mit
b) dimerisierten ungesättigten Fettsäuren der Kettenlänge C14 bis C22,
wobei das molare Verhältnis des eingesetzten Glycerinmonoesters zu den dimerisierten Fettsäuren 1 : 2 bis 1 : 1 beträgt, zu Oligomeren mit einem Oligomerisierungsgrad von 1 bis 20 und 0,5 bis 10,0 Gew.% eines Gemisches der nicht umgesetzten Komponenten
a) Glycerinmonoesters und
b) dimerisierte Fettsäuren
bestehen.

Insbesondere handelt es sich dabei um solche Wachse auf nativer Rohstoffbasis, bei denen das Umsetzungsprodukt zu mindestens 90 Gew.% durch folgende Struktur charakterisiert wird:

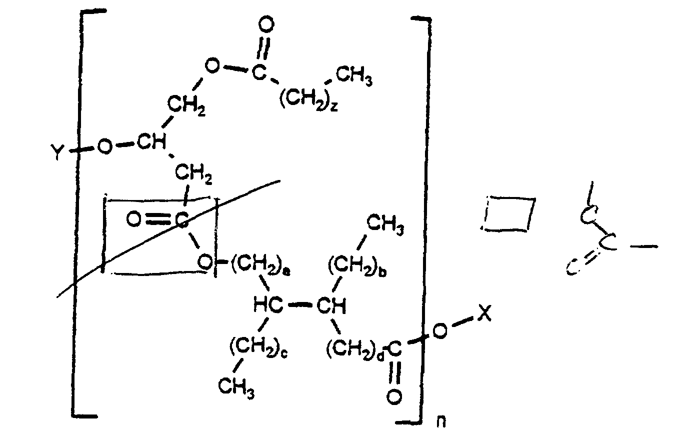

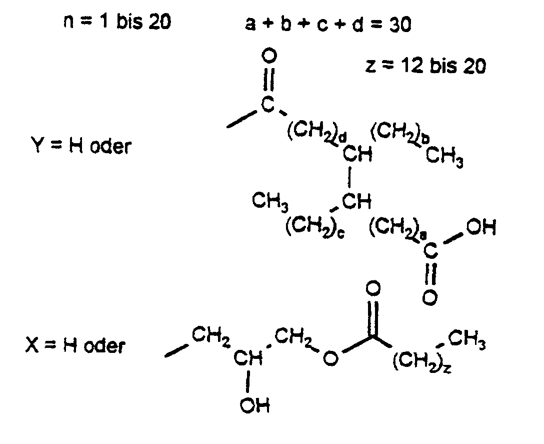

Die besondere Grundstruktur des Oligoglyceryl Dimerates, geprägt durch die sternförmige Anordnung (3- und 4-strahlig), verleiht dem Produkt eine ausgezeichnete Flexibilität, Ölaufnahme und Plastizität.

Zweckmäßig wird der Glycerinmonoester mit gesättigten Fettsäuren gebildet, wobei der Monoesteranteil mindestens 90 % betragen sollte.

Bevorzugt wird als Glycerinmonoester Glycerinmonostearat eingesetzt. Es wird durch Spaltung von Rapsöl mittels Enzymen nach an sich bekannten Verfahren, Isolierung des Glycerinmonooleats und anschließende Hydrierung erhalten. Es kann aber auch ein auf chemischem Wege hergestelltes Glycerinmonostearat mit einem Monoesteranteil von mindestens 90 % zum Einsatz kommen.

Als Einsatzprodukt für die dimerisierte Fettsäure werden zweckmäßig solche mit einem Dimerisierungsgrad von über 75 % und einem Anteil von Trimeren von maximal 15 % eingesetzt.

Bevorzugt wird die dimerisierte Fettsäure aus ungesättigten Fettsäuren gebildet, die durch Splitting von Pflanzenölen gewonnen wurde.

Als Oligomerisierungsgrad wird ein Wert bis 20 angestrebt, vorzugsweise sollte er 2 bis 8 betragen.

Die erfindungsgemäßen Wachse auf nativer Rohstoffbasis werden hergestellt, in dem
a) Glycerinmonoester mit geradkettigen C14 bis C22-Fettsäuren mit
b) dimerisierten ungesättigten Fettsäuren der Kettenlänge C14 bis C22,
wobei das molare Verhältnis der eingesetzten Glycerinmonoester zu den dimeren Fettsäuren 1 : 2 bis 1 : 1 beträgt,
in Gegenwart eines sauren Katalysators bei Temperaturen von 100 ° bis 160 °C, vorzugsweise in der Schmelze, umgesetzt werden.

Als saure Katalysatoren können die üblicherweise zur Veresterung bzw. zu Kondensationsreaktionen angewandten verwendet werden: saure lonenaustauscher, organische Säuren wie Essigsäure, Oxalsäure, Weinsäure oder anorganische Säuren wie Schwefelsäure oder Phosphorsäure.

Die Oligokondensationsreaktion findet vorzugsweise in der Schmelze bei Temperaturen von 100 bis 160 °C in einem heiz- und kühlbaren Rührbehälter unter Vakuum von 0,5 bis 0,3 bar statt oder durch Austreiben des Wassers mittels Inertgasstrom.

Die Reaktionskontrolle erfolgt wie üblich über die Verfolgung der Säurezahlabnahme mittels Säurezahlbestimmung (DIN 53 402).

Nach Erreichen des gewünschten Umsatzgrades kann sich eine alkalische Wäsche zur Produktreinigung bzw. zum Entfernen von Katalysatorspuren anschließen.

Aber auch die Technologie der azeotropen Kondensation - statt der Vakuumverdampfung - führten zu gutem Erfolg. Als Schleppmittel können hier erfindungsgemäß native Öle verwendet werden.

Das Oligomer kann sehr universell sowohl als Rohstoff für kosmetische und pharmazeutische Formulierungen wie für Cremes, Sticks, Suppositorien, als Modellwachsbasis für zahnärztliche Labortechnik, aber auch für technische Anwendungen, z. B. als Wachskomponente für Schmelzkleber, Wachsmalstifte, Kitt- und Dichtungsmassen, und als Beschichtungskomponenten für Nahrungsmittel(verpackungen), z.B. Käsewachs, genutzt werden.

### Ausführungsbeispiele:

### Beispiel 1: Herstellung eines Oligoglycerin Dimerates

In einer mit Außenheizung versehenen Laborglasapparatur (Mehrhalskolben) mit Rührer, Einleitungsrohr für Inertgas, Ableitung für Inertgas und Reaktionswasser und Temperaturmessung werden 270 g Glycerinmonostearat und 200 g Dimersäure in das mit Stickstoff beatmete Reaktionsgefäß eingetragen und auf eine Temperatur von 120 ± 5 °C gebracht. Die eingesetzte Dimersäure ist eine aus ungesättigten Fettsäuren durch Dimerisation gewonnene Polymerfettsäure. Die ungesättigten Fettsäuren werden durch Splitting von pflanzlichen Triglyceriden gewonnen, vorzugsweise sind dies C18-Fettsäuren. Die Dimersäure besteht zu ca. 80 % aus Dimersäure, 15 % Trimersäure und ca. 5 % Monomersäure. Die Rührgeschwindigkeit wird so eingestellt, daß eine gute Durchmischung der Reaktionskomponenten gewährleistet ist. Anschließend wird der Intertgasstrom geregelt und 0,1 ml konzentrierte Schwefelsäure zugesetzt. Der Verlauf der Umsetzung wird über Säurezahlbestimmung geprüft und bis zur Säurezahl von 5 bis 15 mg KOH/g umgesetzt. Hierzu wird in Prüfintervalle eine Probe aus dem Reaktionskolben entnommen.

Das Endprodukt weist folgende allgemeine Wachskenndaten auf:

| **Eigenschaft** | **Einheit** | **Wert** | **Methode** |
|---|---|---|---|
| Säurezahl | mg KOH/g | 13,7 | DIN 53 402 |
| Verseifungszahl | mg KOH/g | 181,0 | DIN 53 401 |
| Erstarrungspunkt | °C | 44,2 | DIN ISO 2207 |
| Nadelpenetration | 1/10 mm | 114,4 | DIN 51 579 |
| Viskosität (100 °C) | mm²/s | 74,1 | DIN 51 562 |

Der Oligomerisationsgrad wird mittels Gelperchromatographie (GPC) unter folgenden Bedingungen ermittelt:
. GPC-Hochtemperaturgerät der Fa. Knauer bei 135 °C
. 1,2,4-Trichlorbenzen als Elutionsmittel (1 ml/min); 0,005 g/l Antioxidans
. Trennsäulen: je 30 cm (ø 7,5 mm) Polystyrengel, 2x 500 Å (CKB) 1x 100 Å (Waters).

Zur Kalibrierung dienen einerseits die Ausgangsprodukte Dimersäure und Glycerinmonostearat und zum anderen die Peakmaxima der niederen Oligomeren bis n=2. Daraus wird eine für die GPC-Methode übliche, lineare Kalibrierfunktion zwischen log M (M = Molmasse)und Elutionsvolumen V abgeleitet und auf höhere Molmassen extrapoliert. Von den höheren Oligomeren wird der Summenanteil ermittelt.

Das hergestellte Produkt besitzt die folgende Zusammensetzung:
. Dimersäure nicht nachweisbar
. Glycerinmonostearat 9,9 %
. Oligomer Dimersäure/Glycerinmonostearat 1 : 1 38,0 %
. Oligomer Dimersäure/Glycerinmonostearat 1 : 2 7,9 %
. Oligomer Dimersäure/Glycerinmonostearat 2 : 1 6,2 %
. Oligomer Dimersäure/Glycerinmonostearat 2 : 2 bis 7 : 7 38 %

### Beispiel 2

Es wird dieselbe Apparatur, wie im Beispiel 1 beschrieben, eingesetzt.
Als Technologie wird eine azeotrope Kondensation benutzt.

| | |
|---|---|
| Einsatzprodukt | 250 g Glyzerinmonomyristat |
| | 180 g Dimersäure |
| Katalysator | 1 ml Phosphorsäure |
| Schleppmittel | 50 ml Terpen |

Als Dimersäure wird eine polymerisierte Fettsäure verwendet, die nach der Dimerisierung gehärtet wurde. Sie weist die folgende Zusammensetzung auf:

| | |
|---|---|
| Monomergehalt (C18) | 1 % |
| Dimergehalt (C36) | 75 % |
| Trimergehalt (C54) | 14 % |

und besitzt eine Säurezahl von 195 mgKOH/g.

Kenndaten des Endproduktes:

| **Eigenschaft** | **Einheit** | **Wert** | **Methode** |
|---|---|---|---|
| Säurezahl | mg KOH/g | 9,7 | DIN 53 402 |
| Verseifungszahl | mg KOH/g | 180 | DIN 53 401 |
| Erstarrungspunkt | °C | 39 | DIN ISO 2207 |
| Nadelpenetration | 1/10 mm | n.b. | DIN 51 579 |
| Viskosität (100 °C) | mm²/s | 55,8 | DIN 51 562 |

Das Oligomere besitzt folgende Zusammensetzung:
. Dimersäure nicht nachweisbar
. Glycerinmonomyristat 4,8 %
. Oligomer Dimersäure/Glycerinmonomyristat 1 : 1 49 %
. Oligomer Dimersäure/Glycerinmonomyristat 1 : 2 11 %
. Oligomer Dimersäure/Glycerinmonomyristat 2 : 1 7,3 %
. Oligomere Dimersäure/Glycerinmonomyristat 2: 2 bis 7 : 7 27,9 %

Nach den Ergebnissen der durchgeführten toxikologischen Untersuchungen ist das Produkt weder toxisch (LD₅₀ p.o. Ratte: > 5000 mg/kg KM) noch haut- oder augenreizend. Es bewirkt weder eine Sensibilisierung der Haut noch ist es mutagen und erfüllt die 155.BGA-Mitteilung.

## Patentansprüche

1. Wachse auf nativer Rohstoffbasis, **dadurch gekennzeichnet, daß** sie zu mindestens 90 Gew.% aus dem Umsetzungsprodukt von
a) Glycerinmonoestern mit geradkettigen C14 bis C22-Fettsäuren mit
b) Dimersäuren aus ungesättigten Fettsäuren der Kettenlänge C14 bis C22,
wobei das molare Verhältnis des eingesetzten Glycerinmonoesters zu den dimerisierten Fettsäuren 1 : 2 bis 1 : 1 beträgt,
zu Oligomeren mit einem Oligomerisierungsgrad von 1 bis 20 und 0,5 bis 10,0 Gew.% eines Gemisches der nicht umgesetzten Komponenten
a) Glycerinmonoesters und
b) Dimersäuren
bestehen.

2. Wachse auf nativer Rohstoffbasis nach Anspruch 1, **dadurch gekennzeichnet, daß** das Umsetzungsprodukt zu mindestens 90 Gew.% durch folgende Struktur charakterisiert wird: 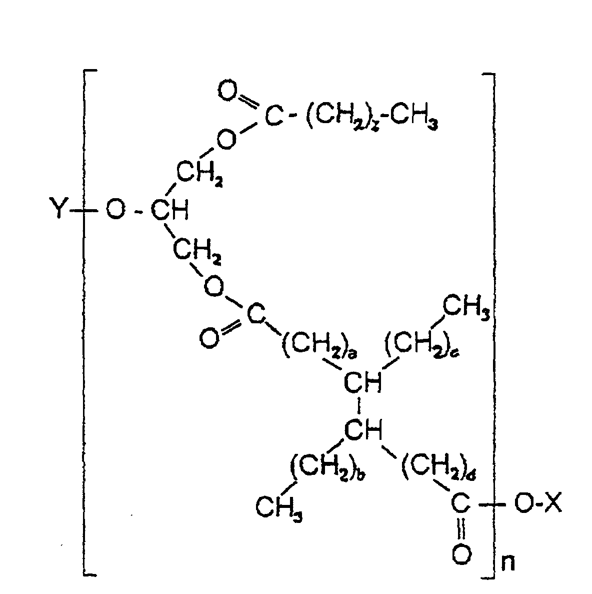 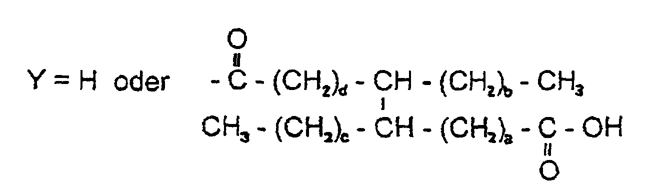 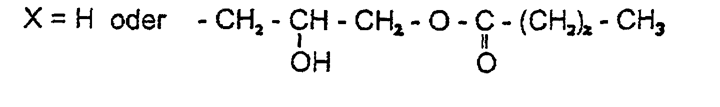
n = 1 bis 20 a+b+c+d = 30 z = 12 bis 20

3. Wachse auf nativer Rohstoffbasis, nach Anspruch 1 und/oder 2, **dadurch gekennzeichnet, daß** der Glycerinmonoester mit gesättigten Fettsäuren gebildet wird und der Monoesteranteil mindestens 90 % beträgt.

4. Wachse auf nativer Rohstoffbasis nach Anspruch 1 bis 3, **dadurch gekennzeichnet, daß** als Glycerinmonoester Glycerinmonostearat eingesetzt wird.

5. Wachse auf nativer Rohstoffbasis nach einem oder mehreren der Ansprüche 1 bis 4, **dadurch gekennzeichnet, daß** die Dimersäure einen Dimerisierungsgrad von über 75 % und einem Anteil von Trimeren von maximal 15 % aufweist.

6. Wachse auf nativer Rohstoffbasis nach einem oder mehreren der Ansprüche 1 bis 5, **dadurch gekennzeichnet, daß** die Dimersäure aus ungesättigten Fettsäuren gebildet ist, die durch Splitting von Pflanzenölen gewonnen wurden.

7. Wachse auf nativer Rohstoffbasis nach einem oder mehreren der Ansprüche 1 bis 6, **dadurch gekennzeichnet, daß** der Oligomerisierungsgrad 2 bis 8 beträgt.

8. Verfahren zur Herstellung von Wachsen auf nativer Rohstoffbasis, **dadurch gekennzeichnet, daß**
a) Glycerinmonoester mit geradkettigen C14 bis C22-Fettsäuren mit
b) Dimersäuren aus ungesättigten Fettsäuren der Kettenlänge C14 bis C22, wobei das molare Verhältnis der eingesetzten Glycerinmonoester zu den dimeren Fettsäuren 1 : 2 bis 1 : 1 beträgt,
in Gegenwart eines sauren Katalysators bei Temperaturen von 100 ° bis 160 °C, vorzugsweise in der Schmelze, umgesetzt wird.

## Claims

1. Waxes based on native raw material **characterized in that** they consist of at least 90 per cent by weight of the products of the conversion of
(a) glycerol monoesters composed of C14 - C22 straight-chain fat acids and
(b) dimeric acids formed from unsaturated fat acids with chain lengths from C14 to C22,
with the molar ratio between the added glycerol monoester and the fat acids after dimerization being 1:2 to 1:1,
to oligomers with the degree of oligomerization being 1 up to 20, and 0.5 per cent up to 10.0 per cent by weight of a mixture comprising the non-converted parts of
(a) the glycerol monoesters, and
(b) the dimeric acids.

2. Waxes based on native raw material according to claim 1, **characterized in that** at least. 90 per cent by weight of the conversion product's structure is as follows: 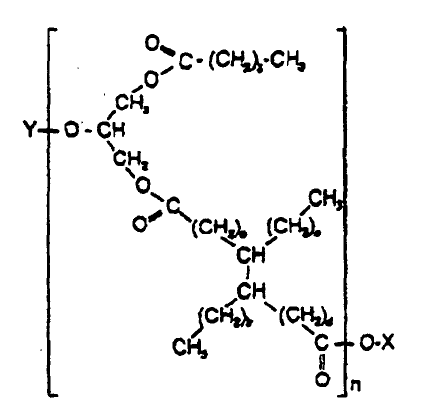 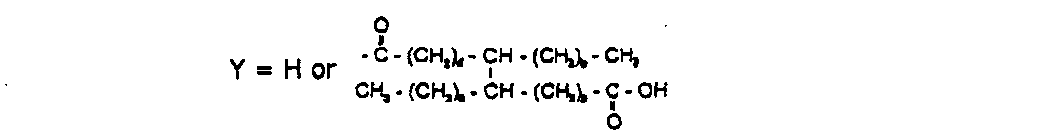 X = H or 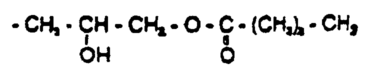
n = 1 through to 20 a+b+c+d = 30 z = 12 through to 20

3. Waxes based on native raw material according to claim 1 and/or 2, **characterized in that** the glycerol monoester is formed from saturated fat acids, and that the monoester's concentration is at least 90 per cent.

4. Waxes based on native raw material according to claims 1 to 3, characterized in that the glycerol monoester is glycerol monostearate.

5. Waxes based on native raw material according to one or more of the claims 1 to 4, **characterized in that** the dimeric acids' degree of dimerization is over 75 per cent, and that the trimers' concentration is max. 15 per cent.

6. Waxes based on native raw material according to one or more of the claims 1 to 5, **characterized in that** the dimeric acid is formed from unsaturated fat acids which are recovered from a vegetable oil splitting process.

7. Waxes based on native raw material according to one or more of the claims 1 to 6, **characterized in that** the degree of oligomerization is 2 up to 8.

8. Method for the preparation of waxes based on native raw material characterized in that
(a) glycerol monoesters composed of C14 - C22 straight-chain fat acids and
(b) dimeric acids formed from unsaturated fat acids with chain lengths from C14 to C22,
with the molar ratio between the added glycerol monoester and the dimeric fat acids being 1:2 to 1:1,
are converted, preferably in melting, in the presence of an acid catalyst at temperatures from 100°C up to 160°C.

## Revendications

1. Cires à base de matières premières natives, **caractérisées par le fait qu'**elles sont constituées au moins de 90 % en poids du produit de transformation de
(a) monoesters de glycérine avec des acides gras en chaînes droites de C14 à C22 avec
(b) des acides dimères d'acides gras insaturés d'une longueur de chaîne de C14 à C 22,
le rapport molaire du monoester de glycérine utilisé étant de 1 : 2 jusqu'à 1 : 1,
en oligomères avec un degré d'oligomérisation de 1 à 20 et de 0,5 à 10,0 % en poids d'un mélange des constituants non transformés de
(a) monoesters de glycérine et
(b) d'acides dimères.

2. Cires à base de matières premières natives selon revendication 1, **caractérisées par le fait que** le produit de transformation de 90 % de poids au moins est dérterminé par la structure suivante: 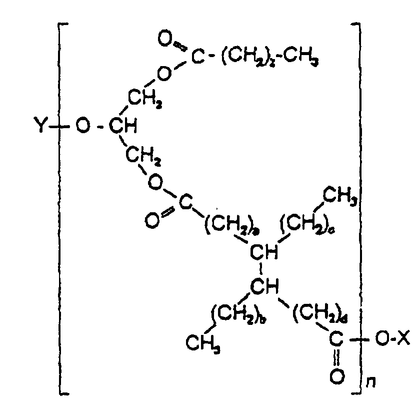 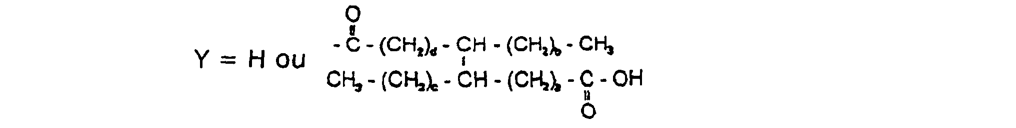 X = H ou 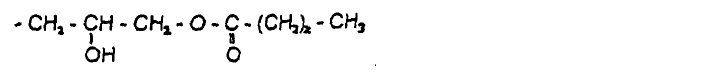
n = 1 jusqu'à 20 a+b+c+d = 30 z = 12 jusqu'à 20

3. Cires à base de matières premières natives selon les revendication 1 et/ou 2, **caractérisées par le fait que** le monoester de glycérine est formé d'acides gras saturés et que la part de monoester est au moins de 90 %.

4. Cires à base de matières premières natives selon les revendications de 1 à 3, **caractérisées par le fait que** du monostéarate de glycérine est utilisé comme monoester de glycérine.

5. Cires à base de matières premières natives selon une ou plusieurs des revendications de 1 à 4, **caractérisées par le fait que** l'acide dimère présente un degré de dimérisation de plus de 75 % et une part de trimères et 15 % au maximum.

6. Cires à base de matières premières natives selon une ou plusieurs des revendications de 1 à 5, **caractérisées par le fait que** l'acide dimère est formé d'acides gras insaturés, obtenus par le splittage d'huiles végétales.

7. Cires à base de matières premières natives selon une ou plusieurs des revendications de 1 à 6, **caractérisées par le fait que** le degré d'oligomérisation est de 2 à 8.

8. Cires à base de matières premières natives, **caractérisées par le fait qu'**est transformé
(a) du monoester de glycérine avec des acides gras en chaînes droites de C14 à C22 avec
(b) des acides dimères d'acides gras insaturés d'une longueur de chaîne de C14 à C 22,
le rapport molaire du monoester de glycérine utilisé avec les acides gras dimères étant de 1 : 2 jusqu'à 1 : 1, en présence d'un calalyseur acide par des températures de 100 jusqu'à 160 °C, de préférence dans la matière fondue.
